# EUROPEAN PATENT SPECIFICATION

(11) **EP 0 475 786 B1**
(45) Date of publication and mention of the grant of the patent: **17.07.1996**
(21) Application number: 91308412.5
(22) Date of filing: 13.09.1991
(51) Int. Cl.: G01N 33/569, G01N 33/58

(54) **Method for determination of antistreptolysin O**
Verfahren zur Bestimmung von Antistreptolysin-O
Procédé pour la détermination de l'antistreptolysin-O

(30) Priority: 13.09.1990 JP 243622/90
(43) Date of publication of application: 18.03.1992
(73) Proprietor: WAKO PURE CHEMICAL INDUSTRIES LTD, Chuo-ku Osaka (JP)
(72) Inventor: Kida, Masaaki, Suita-shi (JP); Yoshikawa, Katsumi, Kadoma-shi (JP); Makiyama, Ryoko, Amagasaki-shi (JP); Takano, Susumu, Nagaokakyo-shi (JP)
(74) Representative: Baillie, Iain Cameron

(56) References cited:
- EP-A- 0 106 370
- EP-A- 0 268 773
- EP-A- 0 382 400

## Description

### BACKGROUND OF THE INVENTION

This invention relates to a method for determination of antistreptolysin O (ASO) value using liposomes.

Determination of ASO value is widely used procedure for the diagnosis of previous hemolytic-streptococcal infection. Streptolysin O (SLO) is a hemolytic toxin produced by hemolytic streptococci and has antigenicity. Therefore, when hemolytic-streptococcal infections occur, antibody against SLO (that is ASO) value is increased, and hence previous hemolytic-streptococcal infections can be confirmed by detecting this increase.

Conventional methods for determining ASO value include the Rantz-Randall method and the micro-titer method which use human (0 group), rabbit or sheep erythrocites. In each of these methods, ASO value is determined by utilizing a so-called neutralization reaction in which SLO as antigen reacts with ASO in a sample serum, resulting in inactivation of the toxic activities such as hemolytic activity and cytolytic activity of ASO. However, in these methods, fresh erythrocytes of an animal or a human being which are very unstable are unavoidably needed, and therefore measured values tend to scatter and a complicated procedure is required. Furthermore, the reaction time is relatively long. Accordingly, the automation of these methods is very difficult.

On the other hand, for solving such problems, a determination method by immuno-nephelometry utilizing a kind of precipitation reaction has also been reported (Japanese Patent Unexamined Publication No. 61-25062, etc.). However, the degree of turbidity produced by the antigen-antibody reaction between SLO and ASO is much lower than that in other antigen-antibody reactions. Therefore, it is necessary to add a so-called agglutination promotor and the like in such a larger amount that they precipitate also other proteins non-specifically. Accordingly, a determination system becomes complicated, accurate determination is very difficult, and the prozone phenomenon tends to occur. Thus, the automation of said determination method involves many problems.

As a method which solves such problems described above, some of the present inventors have developed a novel method for determination of ASO value which can be automated by using liposomes encapsulating a labeling substance, in place of erythrocytes (Japanese Patent Unexamined Publication No. 63-184063 = EP-A 268773). This method does not require employment of an unstable substance such as erythrocytes, and it is simple, permits rapid and accurate determination, and can be automated. But, the method is disadvanteous in that the measuring sensitivity changes depending on production lots of liposomes used, and there has been a desire to seek further improvement in the method. EP-A-0 382 400 and EP-A-0 106 370 both describe methods of liposome production, including a surfactant or detergent removal method.

### SUMMARY OF THE INVENTION

The present invention has been made in consideration of such conditions and is intended to provide a novel method for determination of ASO value which is simple, permits rapid and accurate determination, and can be automated, and the measuring sensitivity of which hardly changes depending on production lots of liposomes.

The present invention provides a method for determination of ASO value comprising using a sample containing ASO, liposomes encapsulating a labeling substance, and streptolysin O (SLO), and measuring the amount of the labeling substance released by lysis of the liposome membrane by the lytic activity of SLO, said liposomes being those prepared by a detergent removal method.

### BRIEF DESCRIPTION OF THE DRAWINGS

Fig. 1 and Fig. 2 show the results obtained in Experimental Example 2. Fig. 1 shows calibration curves for antistreptolysin O (ASO) value which were obtained by using 4 lots of liposomes, respectively, prepared by a detergent removal method. Fig. 2 shows calibration curves for ASO value which were obtained by using 4 lots of liposomes, respectively, prepared by the voltexing method.

Fig. 3 shows the results of examining the reactivity of liposomes kept in cold storage for a predetermined number of days with streptolysin O (SLO) which were obtained in Example 4.

Fig. 4 shows a calibration curve for ASO value obtained in Example 1. Fig. 5 shows the correlation between the determination method of this invention and a conventional method (the Rantz-Randall method) which was determined in Example 1.

Fig. 6 shows a calibration curve for ASO value obtained in Example 2. Fig. 7 shows the correlation between the determination method of this invention and a conventional method (the Rantz-Randall method) which was determined in Example 2.

Figs. 8, 10 and 12 are graphs showing calibration curves for ASO value obtained in Examples 3, 4, and 5, respectively.

Figs. 9, 11 and 13 show the correlation between the determination method of this invention and a conventional method (the Rantz-Randall method) which was determined in Example 3, 4 and 5, respectively.

### DESCRIPTION OF THE PREFERRED EMBODIMENTS

In the course of investigation for solving the problem in a method for determination of ASO value using liposomes encapsulating a labeling substance, in place of erythrocytes, namely, the problem that the measuring sensitivity changes depending on production lots of liposomes, the present inventors noted that properties of liposomes vary depending on the kind of a method for preparing the liposomes. It was considered that the above problem could be solved by choosing the preparation method, and we earnestly investigated. Consequently, it was found that when ASO value is measured by using liposomes prepared by a detergent removal method, the measuring sensitivity hardly changes depending on production lots of liposomes, whereby the present invention was accomplished.

In the present invention, the detergent removal method means a method comprising adding an aqueous surfactant solution to a lipid film formed so as to disperse the film uniformly in the aqueous surfactant solution, and removing the surfactant immediately by dialysis, gel filtration, adsorption on resin, or the like method.

As the main constituent of membrane of the liposome used in this invention, there can be exemplified each or combination of two or more substances used as materials for membrane in preparation of conventional liposomes, i.e., natural lecithins (e.g. egg yoke lecithin, and soybean lecithin) and phospholipids such as dipalmitoylphosphatidylcholine (DPPC), dimyristoylphosphatidylcholine (DMPC), distearoylphosphatidylcholine (DSPC), dioleoylphosphatidylcholine (DOPC), dimyristoylphosphatidylethanolamine (DMPE), phosphatidylglycerol (egg yolk), dipalmitoylphosphatidylglycerol (DPPG), dimyristoylphosphatidic acid (DMPA), dipalmitoylphosphatidic acid (DPPA), palmitoyloleoylphosphatidylcholine (POPC), etc.; mixtures of these substances and cholesterols; and combinations of the mixtures and lipopolysaccharides (LPS). Although either the above-exemplified phospholipids or other phospholipids may be used, there are preferably used natural lecithins (e.g. egg yolk lecithin and soybean lecithin) and phospholipids comprising an unsaturated fatty acid, for example, palmitoyloleoylphosphatidylcholine (POPC) and dioleoylphosphatidylcholine (DOPC). It is preferable to use as one of constituents of membrane of the liposome a compound having a negative charge, for example, LPS, a phosphatidylglycerol such as phosphatidylglycerol (egg yolk), DPPG, etc. or a phosphatidic acid such as DMPA, DPPA, etc., because the use of such a compound enhances the reactivity of the resulting liposome with SLO and hence the measuring sensitivity.

A method for preparing the liposomes used in this invention is explained below in detail.

First, such phospholipids and cholesterols as are described above are dissolved in a suitable organic solvent (e.g. chloroform, an ether or an alcohol), and the resulting solution is concentrated to dryness under reduced pressure and then sufficiently dried under reduced pressure in a desiccator. Subsequently, an aqueous surfactant solution (usually 50 mM to 300 mM, preferably 100 to 200 mM) is added to the lipid film thus formed and the film is uniformly dispersed there-into. The surfactant used here includes, for example, those heretofore often used in the art, such as cholic acid, Tritons (trade names, Rohm and Haas Co.), octyl glucoside, octyl thioglucoside, etc., especially surfactants having a high critical micelle concentration (CMC), such as octyl glucoside, octyl thioglucoside and the like are preferred. Next, if necessary, a lipopolysacctiaride is added in powder form as it is or in solution, followed by adding thereto a solution containing a desired labeling substance, and the resulting mixture is sufficiently stirred. It is most preferable to remove the surfactant immediately after the stirring, and a method for the removal includes per se well-known methods such as dialysis, gel filtration, adsorption on resin, etc. As to the treatment conditions, the treatment time is 1 minute to 24 hours, preferably 30 minutes to 3 hours, and the treatment temperature may be properly chosen in the range of about 0° to about 70°C though it is somewhat varied depending on the constituents of membrane of the liposome, etc. Of the treatment methods exemplified above, dialysis is the most preferable. The liposomes thus obtained are used or stored after being concentrated by ultrafiltration or the like so as to have a predetermined concentration.

When the liposomes used in this invention are prepared in the manner described above, it is preferable to add a water-soluble polymeric compound such as serum albumin (derived from, for example, bovine serum or human serum), water-soluble gelatin, polyethylene glycol, polyvinyl alcohol or the like at the time of adding the surfactant solution or the solution containing the labeling substance to the lipid film obtained, to encapsulate the same in the liposomes together with the labeling substance, because the stability of the liposomes during storage is further improved by the water-soluble polymeric compound. The water-soluble polymeric compound may be added in powder form as it is together with the surfactant solution or the solution containing the labeling substance, or it may be added in the form of a solution prepared by dissolving the compound in the surfactant solution or the solution containing the labeling substance. The amount of the compound added is not critical so long as it does not hinder the preparation of the liposomes. For example, when the compound is added in the form of a solution prepared by dissolving the compound in the solution containing the labeling substance, the concentration of the compound in the solution thus prepared is usually adjusted to 1 to 100 mg/ml, preferably 10 to 30 mg/ml.

The labeling substance to be encapsulated in the liposomes used in this invention is not critical, and any labeling substance may be used so long as it is detectable. Typical examples of the labeling substance are enzymes such as alkaline phosphatase, glucose-6-phosphate dehydrogenase, β-galactosidase, etc.; coenzymes such as nicotinamide-adenine dinucleotide (NAD), nicotinamide-adenine dinucleotide phosphate (NADP), flavin-adenine dinucleotide (FAD), etc.; luminescent compounds such as luminol, luciferin, bis(2,4,6-trichlorophenyl)oxalate, N-methylacridium ester, etc.; compounds which can emit fluorescence, such as carboxyfluorescein, etc.; dyes such as Arsenazo III, 4-(2-pyridylazo)resorcinol, 2-(5-bromo-2-pyridylazo)-5-(N-propyl-N-sulfopropylamino)phenol sodium salt, etc.; sugars such as glucose, etc.; ionic compounds such as potassium dichromate, sodium dichromate, sodium chloride, etc.; radical compounds such as nitroxide compounds, etc.; and substances having properties as spin, which are represented, for example, by 2,2,6,6-tetramethyl-piperidin-1-oxyl (TEMPO).

A method for measuring the amount of the labeling substance is, as a matter of course, varied depending on the kind of the labeling substance used. For example, when the labeling substance is an enzyme, it is measured according to, for exmaple, the method described in Tsunehiro Kitagawa, Toshio Nanbara, Akio Tsuji, and Eiji Ishikawa "Koso Men-eki Sokuteiho", an extra issue No. 31 of Tanpakushitsu Kakusan Koso, pp. 51-63, KYORITSU-SHUPPAN Ltd., published on Sept. 10, 1987, etc. When the labeling substance is a coenzyme, it is measured according to, for example, the method disclosed in U.S. Patent 4,704,355, etc. When the labeling substance is a compound which can emit fluorescence, it is measured according to, for example, the method described in Akira Kawano "Zusetsu Keiko-kotai" 1st ed., Soft Science, Inc., 1983, etc. When the labeling substance is a luminescent compound, it is measured according to, for example, the method described in Tsunehiro Kitagawa, Toshio Nanbara, Akio Tsuji and Eiji Ishikawa "Koso Men-eki Sokuteiho", an extra issue No. 31 of Tanpakushitsu Kakusan Koso, pp. 252-263, KYORITSU-SHUPPAN Ltd., published on Sept. 10, 1987, etc. When the labeling substance is a dye, it is measured according to, for example, the method described in Andrews, Janoff et al., Clin. Chem., 29, 1587 (1983), etc. When the labeling substance is a sugar, it is measured according to, for example, the method described in T. Kataoka et al., Eur. J. Biochem., 21, 80 (1971), etc. When the labeling substance is an ionic compound, it is measured according to, for example, the method described in Y. Umezawa et al., Talanta, 31, 375 (1984), etc. When the labeling substance is a radical compound, it is measured according to, for example, the method described in Wu R, Alving et al., J. Immunal Methods, 9, 165 (1975), etc. When the labeling substance is a substance having properties as spin, it is measured according to, for example, the method described in Tsunehiro Kitagawa, Toshio Nanbara, Akio Tsuji and Eiji Ishikawa "Koso Men-eki Sokuteiho" an extra issue No. 31 of Tanpakushitsu Kakusan Koso, pp. 264-271, KYORITSU-SHUPPAN Ltd., published on Sept. 10, 1987, etc.

The amounts of the liposomes, labeling substance, SLO, etc. used in the determination method of this invention in terms of their concentrations in a total reagents mixture, i.e., a mixture of a sample, a SLO solution, substrate solution and a liposome suspension (or a total reagents mixture before addition of the reaction terminator solution hereinafter described, in the case of determination using the reaction terminator solution) are as follows. The amount of the liposomes is usually 1 to 500 nmol/ml, preferably 5 to 100 nmol/ml, in the total reagents mixture, in terms of the amount of phospholipid. The amount of the labeling substance to be encapsulated in the liposomes is varied depending on the kind of the labeling substance, but for example, when the labeling substance is alkaline phosphatase (hereinafter abbreviated as AP), the amount is usually 0.01 to 0.6 unit/ml, preferably 0.02 to 0.2 unit/ml, in the total reagents mixture. Although the amount of SLO is not critical because of its variation depending on the kind of the liposome, it is usually 5 to 5,000 HU/ml, preferably 50 to 1,000 HU/ml, in the total reagents mixture. HU value of SLO is measured in the following manner.

A mixture of 2 volumes of a 1% rabbit erythrocytes solution and 1 volume of a SLO solution diluted to a predetermined degree is incubated at 37°C for 30 minutes and then centrifuged (3,000 r.p.m. x 5 min), and a degree of dilution of SLO solution at which 100% hemolysis takes place is confirmed, and taken as the HU value of a starting SLO solution.

The amount of a substrate used when an enzyme is used as the labeling substance in the determination method of this invention, is varied depending on the kinds of the enzyme and substrate used, but for example, when AP is used, the amount is usually 0.5 to 10 mM, preferably 2 to 5 mM, in terms of the concentration of p-nitrophenylphosphate as substrate solution.

Buffers used in the determination method of this invention include, for example, phosphoric acid and its salts, boric acid and its salts, tris(hydroxymethyl)aminomethane (Tris), Good's buffers and Veronal but are not limited thereto, and any conventional buffer may be used.

Liquid reagents used in the determination method of this invention may be properly incorporated with reagents usually used in liquid reagents in the art, for example, water-soluble proteins such as bovine serum albumin (BSA), gelatin and the like, sugars, chelating agents, reducing agents and antiseptics. The concentration ranges of these reagents added may be properly selected, for example, from concentration ranges, usually employed in liquid reagents for immunoassay in which a per se well-known antigen-antibody reaction is utilized. Since SLO is relatively unstable in an aqueous solution, it is preferable to incorporate water-soluble proteins such as BSA, gelatin, etc. previously into a buffer solution used for the SLO solution, in order to stabilize SLO. The amount of the protein added is preferably about 0.05 to about 2.0 w/v% in the buffer solution.

The pH's of the liquid reagents used in the determination-method of this invention are properly chosen usually in the range of 5 to 9, preferably 6 to 8. The pH's are adjusted using, for example, the buffers exemplified above.

The determination method of this invention is described below, for example, by taking the case where liposomes encapsualting AP as a labeling substance are used and p-nitrophenylphosphate is used as a substrate.

First, a sample and SLO are mixed and then preincubated for about 1 to about 10 minutes, followed by adding thereto the liposomes encapsulating AP; or a sample, SLO and the liposomes are mixed simultaneously. Then, the resulting mixture is incubated for 5 to 30 minutes, after which p-nitrophenylphosphate as substrate is added and the mixture thus obtained is incubated for another 5 to 30 minutes. Thereafter, the reaction is terminated by addition of a reaction terminator solution (e.g. a NaOH solution or a KOH solution, etc.), and absorbance at 410 nm is measured. In the above procedure, the order of addition of SLO and the liposomes may be reversible. The determination can be carried out also by allowing all the reagents (i.e. SLO, the liposomes and the substrate) to act on a sample at the same time, conducting incubation for 5 to 30 minutes, terminating the reaction by addition of a reaction terminator solution, and then measuring absorbance at 410 nm.

Although any of the methods described above may be employed, the method lastly described in which all the reagents are allowed to act on a sample at the same time is superior because its procedure is simple and the determination can be carried out in a short time.

When an enzyme is used as the labeling substance, it is also possible to determine ASO value by measuring the change of absorbance per unit time after preincubating the total reagents mixture for a predetermined time or preincubating a mixture of a sample, a SLO solution and a liposome suspension for a predetermined time and then adding the substrate.

The determination method of this invention is sufficiently applicable not only to a manual method but also to determination using an automatic analyzer, and permits easy and rapid determination. When the determination is carried out by means of an automatic analyzer, combination of reagents, etc. are not critical and there may be properly selected a combination of reagents which seems to be the most suitable in consideration of the type of the automatic analyzer or other factors.

The present invention is characterized in that liposomes prepared by a detergent removal method are used in a method for determination of ASO value using liposomes. The present invention is markedly effective in that by the use of the thus prepared liposomes, it has solved the problem in conventional methods for determination of ASO value using liposomes, namely, the problem that the measuring sensitivity changes depending on production lots of liposomes. The effect obtained by this invention cannot be obtained by using liposomes prepared by the vortexing method, the reverse-phase evaporation method (the REV method) or the like which are generally used as preparation method of liposomes. Although the reason is not clear, it can be presumed to be, for example, as follow.

The method for determination of ASO value of this invention utilizes the lysis of the liposomes by the lytic activity of SLO, and it is conjectured that the rate of lysis of the liposomes by SLO varies depending on the membrane structure (a monolayer, a double layer, a multilayer or the like) of the liposomes and their particle size. Therefore, the following conjecture can be given. According to a detergent removal method, there can always be obtained liposomes which are uniform in their membrane structure and substantially uniform in their particle size, and hence the rate of lysis of the liposomes obtained by said method by the lytic activity of SLO hardly changes depending on production lots of liposomes, so that the measuring sensitivity for ASO value hardly changes depending on production lots of liposomes. On the other hand, according to the voltexing method, the reverse-phase evaporation method (the REV method) or the like, liposomes whose membrane structure is different in different production lots are obtained in an unspecified ratio, and they are widely various in their particle size. Therefore, the rate of lysis of the liposomes obtained by any of these methods by the lytic activity of SLO changes depending on production lots of liposomes, so that the measuring sensitivity for ASO value changes depending on production lots of liposomes.

The method of the present invention can be carried out by using a kit comprising (A) a suspension of liposomes encapsulating a labelling substance and a water soluble polymeric compound, and (B) a solution of SLO, said liposomes being prepared by a detergent removal method. More concretely, the liposome preferably comprises as constituents of membrane at least one phospholipid comprising an unsaturated fatty acid, and a lipid having a negative charge. Further, the kit can further contain a substrate solution. In addition, the liposome may contain a lipopolysaccharide (LPS) as a part of its constituents.

The present invention is illustrated below in further detail with reference to Referential Examples, Experimental Examples and Examples.

### Referential Example 1

### Preparation of liposomes by the voltexing method

In a round bottom flask were placed 2 ml of a 40 mM solution of egg yolk lecithin in chloroform and 2 ml of a 40 mM solution of cholesterol in chloroform, and mixed. Then, the solvent was distilled off by means of a rotary evaporator, followed by vacuum drying in a desiccator for about 2 hours, whereby a thin film of the lipids was formed on the interior surface of the flask. To the film was added 250 µl of an aqueous AP solution [a solution of 2,000 U/ml of AP in 0.01 M N-2-hydroxyethylpiperazine-N′-2-ethanesulfonic acid (HEPES) buffer (pH 7.4)], and the resulting mixture was stirred with a Vortex mixer until it became homogeneous. Then, the mixture was ultracentrifuged (4°C, x 100,000 G, 60 min), after which the supernatant was removed and the pellet thus obtained was suspended in 5 ml of 0.01 M HEPES buffer (pH 7.4) to obtain a liposome suspension.

The above procedure was repeated 4 times, whereby suspensions of 4 lots of liposomes, respectively, were obtained.

### Referential Example 2

### Preparation of liposomes by the REV method

In a round bottom flask were placed 0.325 ml of a 20 mM solution of egg yolk lecithin in chloroform and 0.325 ml of a 20 mM solution of cholesterol in chloroform, and mixed. Then, the solvent was distilled off by means of a rotary evaporator, followed by vacuum drying in a desiccator for about 2 hours, whereby a thin film of the lipids was formed on the interior surface of the flask. To the film were added 0.5 ml each of chloroform and diethyl ether to dissolve the film, followed by adding thereto 80 µl of an aqueous AP solution [a solution of 2,000 U/ml of AP in 0.01 M HEPES buffer (pH 7.4)]. The resulting mixture was vigorously stirred with a Vortex mixer to obtain an emulsion. Subsequently, the emulsion was placed in a rotary evaporator and the solvent was distilled off on a water bath at 43° to 48°C, after which 1 ml of 0.01 M HEPES buffer (pH 7.4) was added to the gel thus obtained, and the resulting mixture was stirred with a Vortex mixer until it became homogeneous. Then, the homogeneous mixture was ultracentrifuged (4°C, x 100,000 G, 40 min x 5), after which the supernatant was removed and the pellet thus obtained was suspended in 2 ml of 0.01 M HEPES buffer (pH 7.4) to obtain a liposome suspension.

The above procedure was repeated 3 times, whereby suspensions of 3 lots of liposomes, respectively, were obtained.

### Referential Example 3

### Preparation of liposomes by a detergent removal method-1

In a round bottom flask were placed 2 ml of a 40 mM solution of egg yolk lecithin in chloroform and 2 ml of a 40 mM solution of cholesterol in chloroform, and mixed. Then the solvent was distilled off by means of a rotary evaporator, followed by vaccum drying in a desiccator for about 2 hours, whereby a thin film of the lipids was formed on the interior surface of the flask. To the film were added 1.2 ml of a 200 mM aqueous n-octyl glucoside solution and 1.8 ml of 0.01 M HEPES buffer (pH 7.4), and the resulting mixture was stirred with a Vortex mixer until it became homogeneous. Subsequently, 250 µl of an aqueous AP solution [a solution of 2,000 U/ml of AP in 0.01 M HEPES buffer (pH 7.4)] was added and stirred for a while. The resulting mixture was placed in a dialyzing tube and dialyzed against 0.01 M HEPES buffer (pH 7.4) at 50°C for 1 hour, after which the liposome suspension thus obtained was ultracentrifuged (4°C, x 100,000G, 60 min) and the supernatant was removed. The pellet thus obtained was suspended again in 5 ml of 0.01 M HEPES buffer (pH 7.4) to obtain a liposome suspension.

### Referential Example 4

### Preparation of liposomes by a detergent removal method-2

In a round bottom flask were placed 2 ml of a 40 mM solution of egg yolk lecithin in chloroform and 2 ml of a 40 mM solution of cholesterol in chloroform, and mixed. Then, the solvent was distilled off by means of a rotary evaporator, followed by vacuum drying in a desiccator for about 2 hours, whereby a thin film of the lipids was formed on the interior surface of the flask. To the film were added 1.2 ml of a 200 mM aqueous n-octyl glucoside solution (containing 6.6 mg/ml of LPS) and 1.8 ml of 0.01 M HEPES buffer (pH 7.4), and the resulting mixture was stirred with a Vortex mixer until it became homogeneous. Subsequently, 250 µl of an aqueous AP solution [a solution of 2,000 U/ml of AP in 0.01 M HEPES buffer (pH 7.4)] was added and stirred for a while. The resulting mixture was placed in a dialyzing tube and dialyzed against 0.01 M HEPES buffer (pH 7.4) at 50°C for 1 hour, after which the liposome suspension thus obtained was ultracentrifuged (4°C, x 100,000G, 60 min) and the supernatant was removed. The pellet thus obtained was suspended again in 5 ml of 0.01 M HEPES buffer (pH 7.4) to obtain a liposome suspension.

The above procedure was repeated 4 times, whereby suspension of 4 lots of liposomes, respectively, were obtained.

### Referential Example 5

### Preparation of liposomes by a detergent removal method-3

In a round bottom flask were placed 2 ml of a 40 mM solution of egg yolk lecithin in chloroform, 2 ml of a 40 mM solution of cholesterol in chloroform and 0.47 ml of a 17 mM solution of phosphatidylglycerol (egg yolk) in chloroform, and mixed. Then, the solvent was distilled off by means of a rotary evaporator, followed by vacuum drying in a desiccator for about 2 hours, whereby a thin film of the lipids was formed on the interior surface of the flask. To the film were added 1.2 ml of a 200 mM aqueous n-octyl glucoside solution and 1.8 ml of 0.01 M HEPES buffer (pH 7.4), and the resulting mixture was stirred with a Vortex mixer until it became homogeneous. Subsequently, 250 µl of an aqueous AP solution [a solution of 2,000 U/ml of AP in 0.01 M HEPES buffer (pH 7.4)] was added and stirred for a while. The resulting mixture was placed in a dialyzing tube and dialyzed against 0.01 M HEPES buffer (pH 7.4) at 50°C for 1 hour, after which the liposome suspension thus obtained was ultracentrifuged (4°C, x 100,000G, 60 min) and the supernatant was removed. The pellet thus obtained was suspended again in 5 ml of 0.01 M HEPES buffer (pH 7.4) to obtain a liposome suspension.

### Referential Example 6

### Preparation of liposomes by a detergent removal method-4

In a round bottom flask were placed 2 ml of a 40 mM solution of egg yolk lecithin in chloroform and 2 ml of a 40 mM solution of cholesterol in chloroform, and mixed.

Then, the solvent was distilled off by means of a rotary evaporator, followed by vacuum drying in a desiccator for about 2 hours, whereby a thin film of the lipids was formed on the interior surface of the flask. To the film were added 1.2 ml of a 200 mM aqueous n-octyl glucoside solution (containing 6.6 mg/ml of LPS) and 1.8 ml of 0.01 M HEPES buffer (pH 7.4), and the resulting mixture was stirred with a Vortex mixer until it became homogeneous. Subsequently, 250 µl of an aqueous AP solution [a solution of 2,000 U/ml of AP in 0.01 M HEPES buffer (pH 7.4) containing 20 mg/ml of each of various predetermined water-soluble polymeric compounds as shown in Table 2] was added and stirred for a while. The resulting mixture was placed in a dialyzing tube and dialyzed against 0.01 M HEPES buffer (pH 7.4) at 50°C for 1 hour, and the liposome suspension thus obtained was ultracentrifuged (4°C, x 100,000G, 60 min), after which the supernatant was removed and the pellets thus obtained was suspended again in 5 ml of 0.01 M HEPES buffer (pH 7.4). Thus, there were obtained various liposome suspensions containing the various water-soluble polymeric compounds, respectively, encapsulated in liposomes (see Table 2).

### Referential Example 7

### Preparation of liposomes by a detergent removal method-5

In a round bottom flask were placed 2 ml of a 40 mM solution of egg yolk lecithin in chloroform, 2 ml of a 40 mM solution of cholesterol in chloroform, and 0.47 ml of a 17 mM solution of phosphatidylglycerol (egg yolk) in chloroform and mixed.

Then, the solvent was distilled off by means of a rotary evaporator, followed by vacuum drying in a desiccator for about 2 hours, whereby a thin film of the lipids was formed on the interior surface of the flask. To the film were added 1.2 ml of a 200 mM aqueous n-octyl glucoside solution (containing 6.6 mg/ml of LPS) and 1.8 ml of 0.01 M HEPES buffer (pH 7.4), and the resulting mixture was stirred with a Vortex mixer until it became homogeneous. Subsequently, 250 µl of an aqueous AP solution [a solution of 2,000 U/ml of AP in 0.01 M HEPES buffer (pH 7.4) containing 20 mg/ml of BSA] was added and stirred for a while. The resulting mixture was placed in a dialyzing tube and dialyzed against 0.01 M HEPES buffer (pH 7.4) at 50°C for 1 hour, and the liposome suspension thus obtained was ultracentrifuged (4°C, x 100,000G, 60 min), after which the supernatant was removed and the pellet thus obtained was suspended again in 5 ml of 0.01 M HEPES buffer (pH 7.4). Thus, there were obtained liposome suspension containing BSA encapsulated in liposomes.

### Experimental Example 1

### Examination (1) of the reactivity of liposomes

### obtained by each of different preparation methods with SLO

With 50 mM Tris buffer (pH 7.8), 10 µl of each of the liposome suspensions obtained in Referential Example 2 and Referential Example 4 was diluted to a volume of 200 µl, and a 2 mM p-nitrophenylphosphate solution (containing 220 HU/ml of SLO) was added. The resulting mixture was incubated at 37°C for 30 minutes, after which the reaction was terminated by addition of 1.5 ml of a 0.1 N NaOH solution, and absorbance at 410 nm of the reaction mixture was measured.

The results obtained are shown in Table 1.

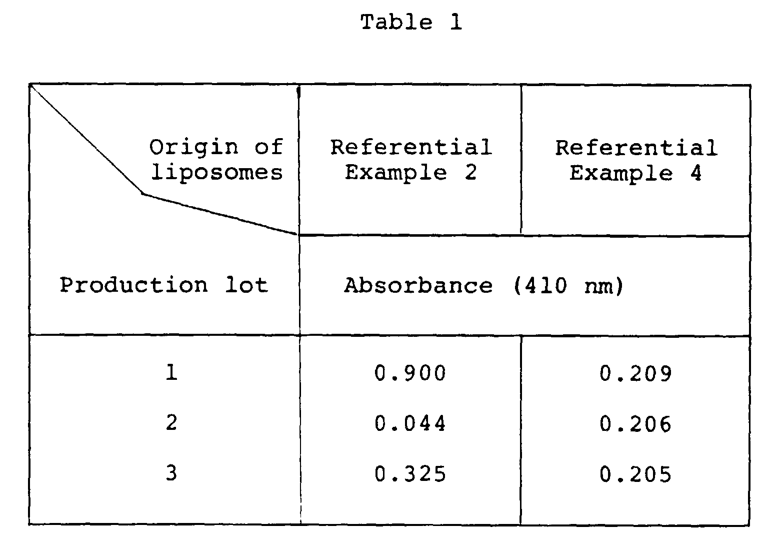

From the results shown in Table 1, it can be seen that the reactivity of the liposomes obtained by the method of Referential Example 2 (the REV method) with SLO changes greatly, depending on production lots of liposomes, and that the reactivity of the liposomes obtained by the method of Referential Example 4 (the detergent removal method) with SLO hardly changes depending on production lots of liposomes.

### Experimental Example 2

### Examination (2) of the reactivity of liposomes obtained by each of different preparation methods with SLO

Each of the liposome solutions obtained in Referential Example 1 or Referential Example 4 was diluted in 11 times with 50 mM Tris buffer (pH 7.8), and 5 µl of an ASO solution having a predetermined concentration was added to the dilution. The resulting mixture was incubated at 37°C for 5 minutes, after which 150 µl of a SLO solution (200 HU/ml) and 150 µl of 2 mM p-nitrophenylphosphate were added. The mixture thus obtained was incubated at 37°C for 5 minutes, and the change of absorbance at 410 nm per unit time of the reaction mixture was measured.

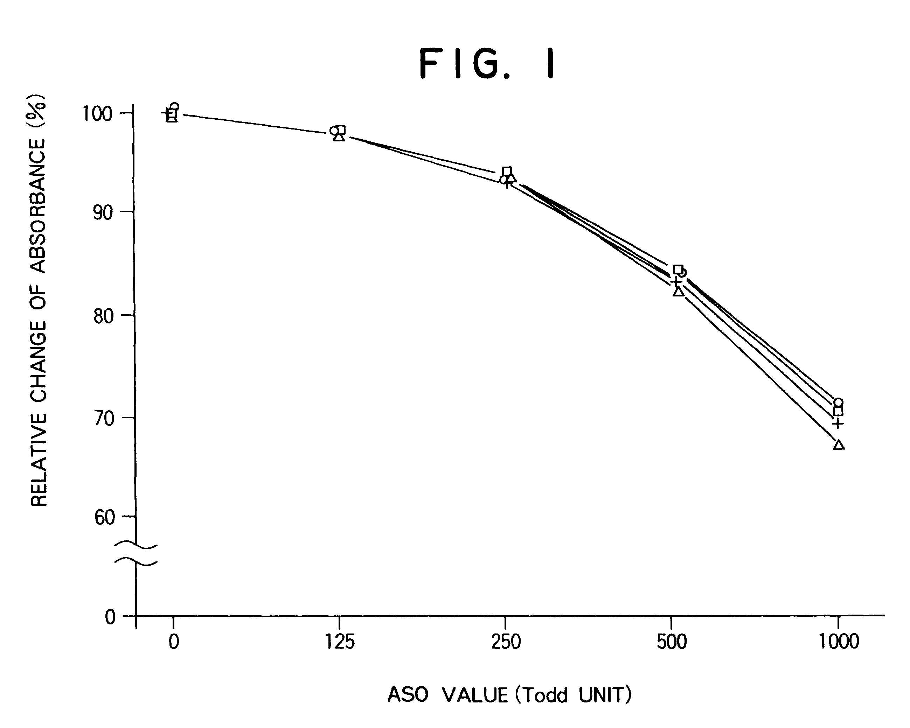

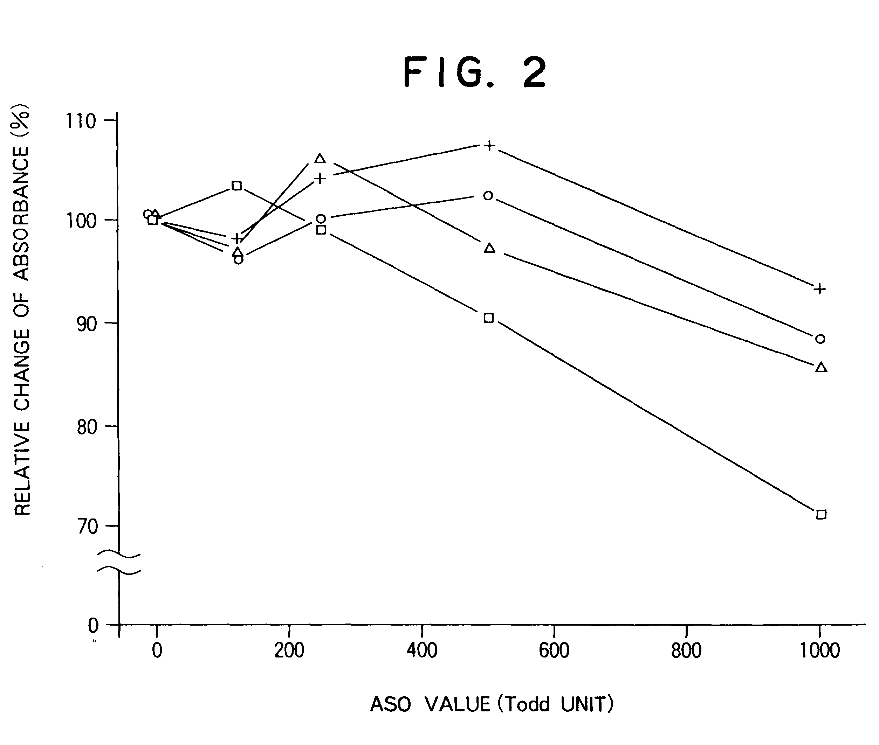

Fig. 1 shows calibration curves obtained by using the liposome suspensions (individual 4 lots) obtained in Referential Example 4. Fig. 2 shows calibration curves obtained by using the liposome suspensions (individual 4 lots) obtained in Referential Example 1. In Fig. 1 and Fig. 2, the axis of abscissa refers to ASO value (Todd) in the ASO solution and the axis of ordinate to the relative change of absorbance (a change of absorbance at an ASO value of 0 in Todd unit was taken as 100).

From the results shown in Fig. 1, it can be seen that the reactivity of the liposomes obtained by the method of Referential Example 4 (the detergent removal method) with SLO hardly changes depending on production lots of liposomes. From the results shown in Fig. 2, it can be seen that the reactivity of the liposomes obtained by the method of Referential Example 1 (the voltexing method) with SLO changes greatly, depending on production lots of liposomes.

### Experimental Example 3

The average particle size of liposomes in each of the liposome suspensions obtained in Referential Example 4 and Referential Exampel 6 was measured by the laser dynamic light scattering method [measuring apparatus: LPA 3000/3100, mfd. by OTSUKA ELECTRONICS CO., LTD.].

The results obtained are shown in Table 2.

**Table 2**

| Origin of liposomes | Water-soluble polymeric compound | Average particle size (nm) | SD (nm) |
|---|---|---|---|
| Referential Example 4 | None | 513 | ±188 |
| Referential Example 6 | BSA | 446 | ±60 |
| Referential Example 6 | Gelatin | 502 | ±74 |
| Referential Example 6 | PEG 20000 | 405 | ±67 |
| Referential Example 6 | PVA | 314 | ±92 |

From the results shown in Table 2, it can be seen that liposomes more uniform in particle size can be obtained by adding a water-soluble polymeric compound in the preparation of the liposomes.

### Experimental Example 4

### Examination of the stability of liposomes during storage

Each of the liposome suspensions obtained in Referential Example 4 and Referential Example 6 (water-soluble polymeric compound: BSA) was kept in cold storage for various predetermined numbers of days. Then, 10 µl of each liposome suspension thus stored was diluted to a volume of 200 µl with 50 mM Tris buffer (pH 7.8), and 400 µl of a 2 mM p-nitrophenylphosphate solution (containing 88 HU/ml of SLO) was added to the dilution. The resulting mixture was incubated at 37°C for 30 minutes, after which the reaction was terminated by addition of 1.5 ml of a 0.1 N NaOH solution, and absorbance at 410 nm of the reaction mixture was measured.

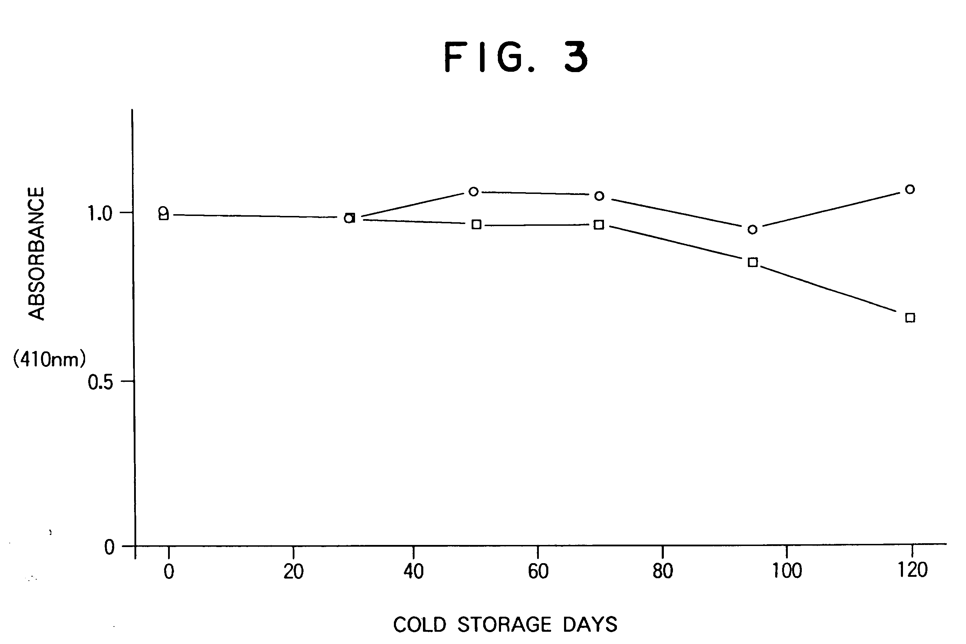

The results obtained are shown in Fig. 3. In Fig. 3, □ shows results obtained for the liposome suspension prepared in Referential Example 4, and ○ shows results obtained for the liposome suspension prepared in Referential Example 6. In Fig. 3, the axis of abscissa refers to cold storage days and the axis of ordinate to absorbance at 410 nm.

From the results shown in Fig. 3, it can be seen that liposomes whose reactivity with SLO does not change for a long period of time can be obtained by adding the water-soluble polymeric compound in the preparation of the liposomes.

### Example 1

ASO value was determined by means of an Autoanalyzer Hitachi 7150.

First, 5 µl of a sample was mixed with 250 µl of a first reagent R1 prepared by diluting the liposome suspension obtained in Referential Example 4 in 20 times with 50 mM HEPES buffer (pH 7.4), and correction blank was conducted. After 5 minutes, 150 µl of a second reagent [50 mM HEPES buffer (pH 7.4) containing 150 HU/ml of SLO and 2 mM p-nitrophenylphosphate] was added. After another 5 minutes, the change of absorbance per minute at 415 nm/700 nm was measured.

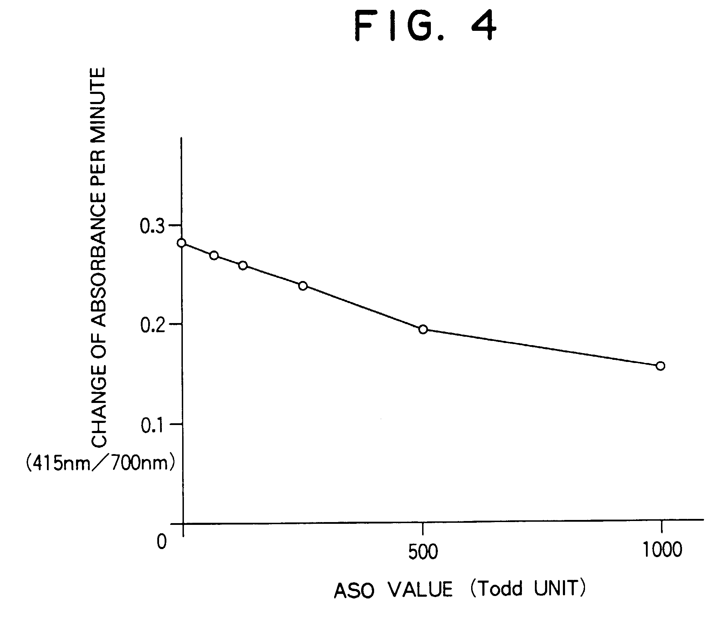

A calibration curve for ASO value in the present method is shown in Fig. 4. In Fig. 4, the axis of abscissa reffers to ASO value (Todd) and the axis of ordinate to the change of absorbance per minute at 415 nm/700 nm.

From the results shown in Fig. 4, it can be seen that a satisfactory calibration curve can be obtained by the method of this invention.

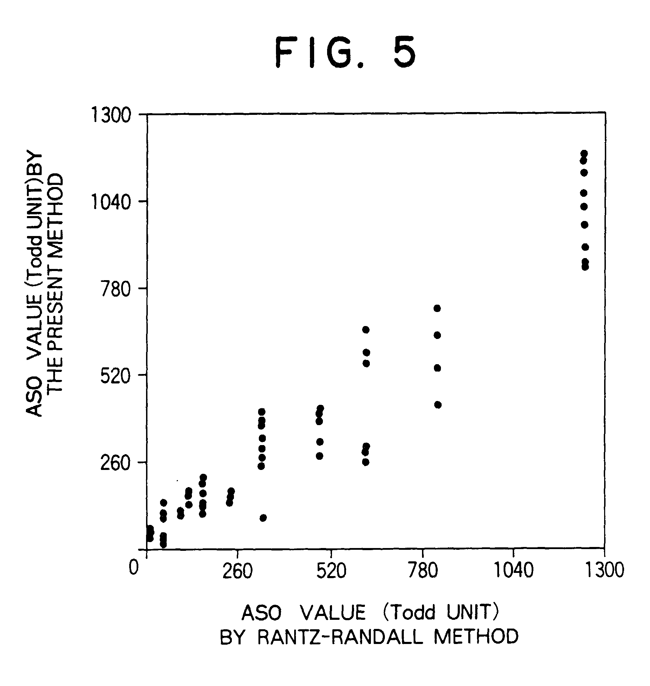

The correlation between the present method and a conventional method (the Rantz-Randall method) is shown in Fig. 5.

From the results shown in Fig. 5, it can be seen that the measured values obtained by the method of this invention are in good correlation with those obtained by the conventional method.

### Example 2

ASO value was measured by means of an Autoanalyzer Cobas Mira (a trade name, mfd. by F. Hoffmann-La Roche & Co. A.G.).

First, 2 µl of a sample was mixed with 80 µl of 50 mM HEPES buffer (pH 7.4) and 160 µl of a first reagent R1 [50 mM HEPES buffer (pH 7.4) containing 200 HU/ml of SLO and 2 mM p-nitrophenylphosphate], and correction blank was conducted. After 5 minutes, 80 µl of a second reagent prepared by diluting the liposome suspension obtained in Referential Example 4 in 101 times with 50 mM HEPES buffer (pH 7.4) was added. After another 15 minutes, absorbance at 415 nm was measured.

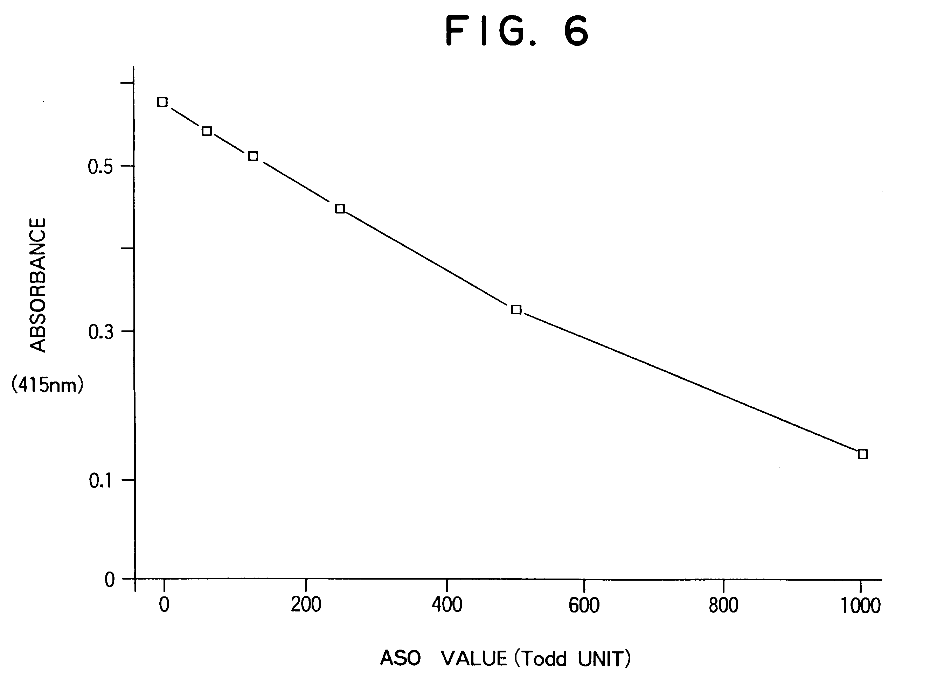

A calibration curve for ASO value in the present method is shown in Fig. 6. In Fig. 6, the axis of abscissa refers to ASO value (Todd) and the axis of ordinate to absorbance at 415 nm.

From the results shown in Fig. 6, it can be seen that a satisfactory calibration curve can be obtained by the method of this invention.

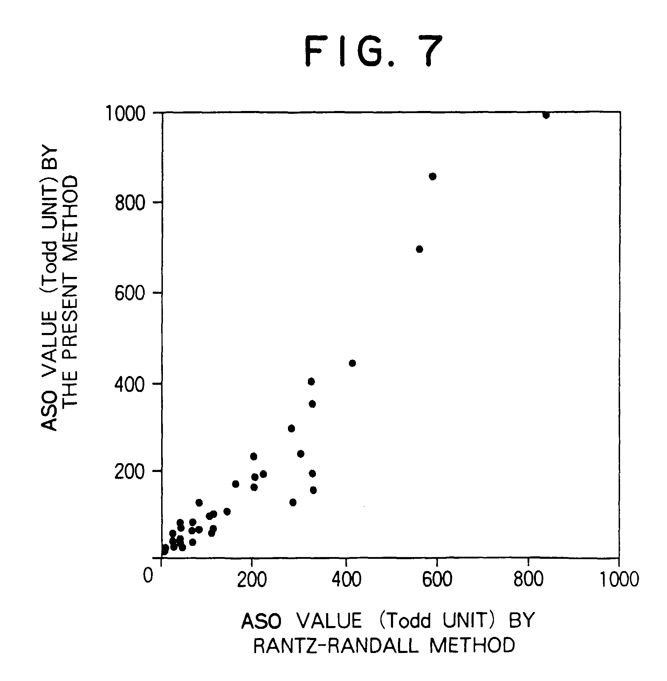

The correlation between the present method and a conventional method (the Rantz-Randall method) is shown in Fig. 7.

From the results shown in Fig. 7, it can be seen that the measured values obtained by the method of this invention are in good correlation with those obtained by the conventional method.

### Example 3

ASO value was determined by means of an Autoanalyzer Hitachi 7150.

First, 5 µl of a sample was mixed with 250 µl of a first reagent R1 prepared by diluting the liposome suspension obtained in Referential Example 5 in 20 times with 50 mM HEPES buffer (pH 7.4), and correction blank was conducted. After 5 minutes, 150 µl of a second reagent [50 mM HEPES buffer (pH 7.4) containing 150 HU/ml of SLO and 2 mM p-nitrophenylphsophate] was added. After-another 5 minutes, the change of absorbance per minute at 415 nm/700 nm was measured.

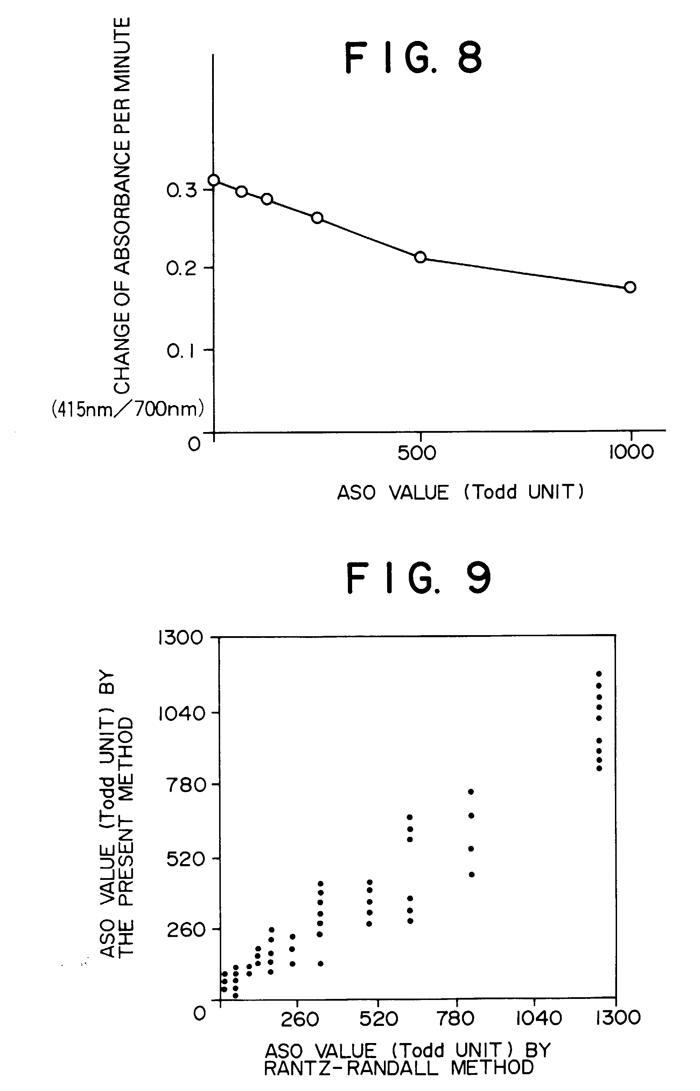

A calibration curve for ASO value in the present method is shown in Fig. 8. In Fig. 8, the axis of abscissa refers to ASO value (Todd) and the axis of ordinate to the change of absorbance per minute at 415 nm/700 nm.

From the results shown in Fig. 8, it can be seen that a satisfactory calibration curve can be obtained by the method of this invention.

The correlation between the present method and a conventional method (the Rantz-Randall method) is shown in Fig. 9.

From the results shown in Fig. 9, it can be seen that the measured values obtained by the method of this invention are in good correlation with those obtained by the conventional method.

### Example 4

ASO value was determined by means of an Autoanalyzer Hitachi Model 7150.

First, 5 µl of a sample was mixed with 250 µl of a first reagent R1 prepared by diluting the liposome suspension (encapsulated BSA) obtained in Referential Example 6 in 20 times with 50 mM HEPES buffer (pH 7.4), and correction blank was conducted. After 5 minutes, 150 µl of a second reagent [50 mM HEPES buffer (pH 7.4) containing 150 HU/ml of SLO and 2 mM p-nitrophenyl-phosphate] was added. After another 5 minutes, the change of absorbance per minute at 415 nm/700 nm was measured.

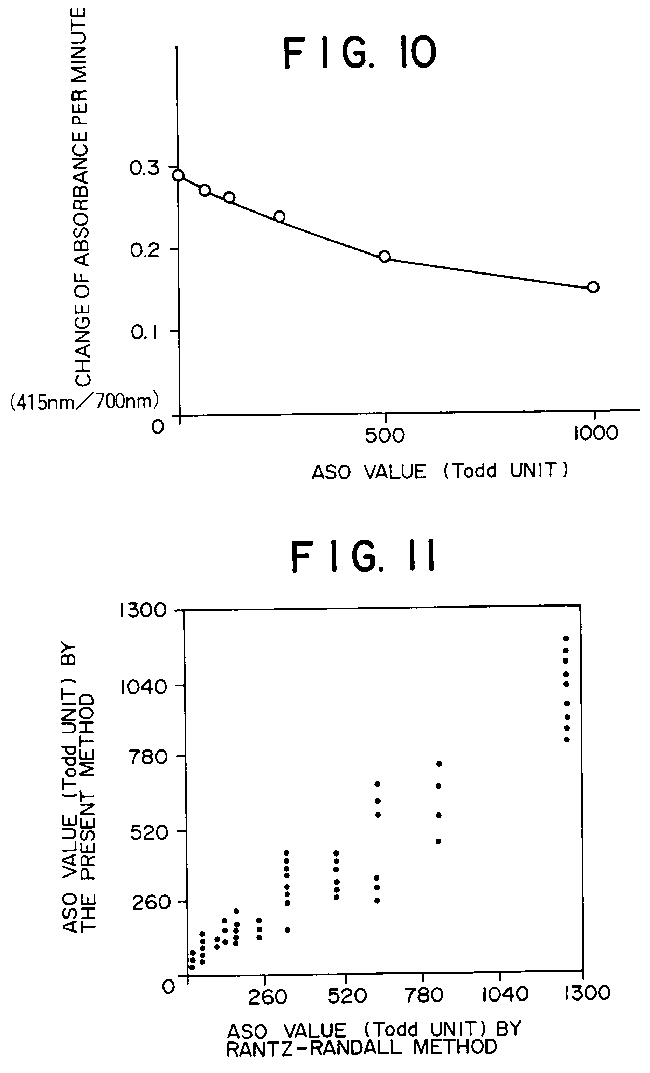

A calibration curve for ASO value in the present method is shown in Fig. 10. In Fig. 10, the axis of abscissa refers to ASO value (Todd) and the axis of ordinate to the change of absorbance per minute at 415 nm/700 nm.

From the results shown in Fig. 10, it can be seen that a satsifactory calibration curve can be obtained by the method of this invention.

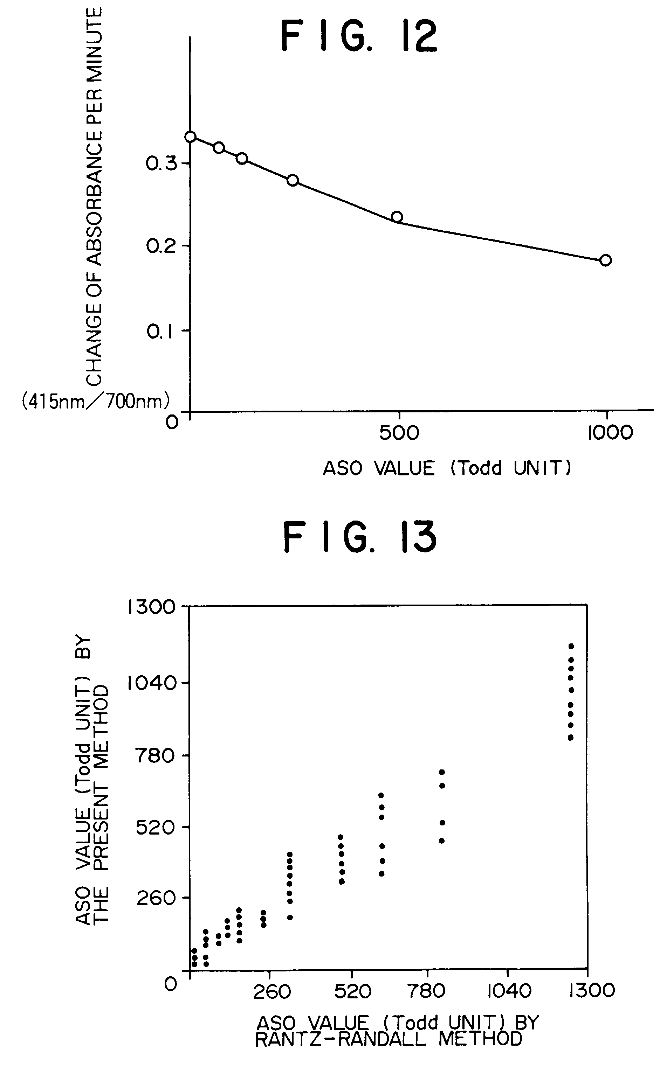

The correlation between the present method and a conventional method (the Rantz-Randall method) is shown in Fig. 11.

From the results shown in Fig. 11, it can be seen that the measured values obtained by the method of this invention are in good correlation with those obtained by the conventional method.

### Example 5

ASO value was determined by means of an Autoanalyzer Hitachi Model 7150.

First, 5 µl of a sample was mixed with 250 µl of a first reagent R1 prepared by diluting the liposome suspension obtained in Referential Example 7 in 20 times with 50 mM HEPES buffer (pH 7.4), and correction blank was conducted. After 5 minutes, 150 µl of a second reagent [50 mM HEPES buffer (pH 7.4) containing 150 HU/ml of SLO and 2 mM p-nitrophenylphosphate] was added. After another 5 minutes, the change of absorbance per minute at 415 nm/700 nm was measured.

A calibration curve for ASO value in the present method is shown in Fig. 12. In Fig. 12, the axis of abscissa refers to ASO value (Todd) and the axis of ordinate to the change of absorbance per minute at 415 nm/700 nm.

From the results shown in Fig. 12, it can be seen that a satisfactory calibration curve can be obtained by the method of this invention.

The correlation between the present method and a conventional method (the Rantz-Randall method) is shown in Fig. 13.

From the results shown in Fig. 13, it can be seen that the measured values obtained by the method of this invention are in good correlation with those obtained by the conventional method.

As described above, the present invention is markedly effective in that in a method for determination of ASO value using liposomes, the problem caused when ASO value is determined using liposomes prepared by a conventional method such as the vortexing method or the REV method, namely, the problem that the measuring sensitivity changes greatly, depending on production lots of liposomes, is solved by use of liposomes prepared by a detergent removal method. Therefore, the present invention contributes greatly to the art.

## Claims

1. A method for the determination of an antistreptolysin 0 value in a sample which comprises adding to said sample containing antistreptolysin 0
(i) a solution containing liposomes encapsulating a labelling substance and
(ii) a solution containing steptolysin 0, and measuring the amount of said labelling substance released by lysis of the liposome membrane by the lytic activity of steptolysin 0, said liposomes being prepared by a detergent removal method.

2. The method according to claim 1, wherein the liposome comprises as constituents of membrane at least one phospholipid comprising an unsaturated fatty acid, and a lipid having a negative charge.

3. The method according to any preceding claim wherein the liposome encapsulates a water-soluble polymeric compound in addition to the labelling substance.

4. The method according to any preceding claim wherein the liposome encapsulates a water-soluble macromolecule in addition to the labelling substance.

5. The method according to any preceding claim wherein the labelling substance is an enzyme, a coenzyme, a luminescent compound, a compound which can emit fluorescence, a dye, a sugar, an ionic compound, a radical compound, or a substance having properties as spin labels.

6. The method according to any preceding claim wherein the main constituent of the membranes of said liposomes is selected from natural lecithins, phospholipids, mixtures of natural lecithins and phospholipids, mixtures of natural lecithins phospholipids and cholesterols and mixtures of natural lecithins phospholipids and lipopolysaccharides.

7. The method of any preceding claim wherein said water-soluble polymeric compound is selected from serum albumin, water-soluble gelatin, polyethylene glycol and polyvinyl alcohol.

8. The method of any one of preceding claims 2 to 7 wherein said phospholipid comprising an unsaturated fatty acid is selected from palmitoyloleoylphosphatidylcholine (POPC) and dioleoylphosphatidylcholine (DOPC) and wherein said lipid having a negative charge is selected from lipopolysaccharides phosphatidylglycerols and phosphatidic acids.

9. A kit for determining the antistreptolysin 0 value in a sample, which comprises (A) a suspension of liposomes prepared by a detergent removal method and encapsulating a labelling substance and a water-soluble polymeric compound, and (B) a solution of streptolysin 0.

10. The kit according to claim 9 wherein each liposome comprises as constituents of its membrane at least one phospholipid comprising an unsaturated fatty acid and a lipid having a negative charge.

11. The kit according to any one of preceding claims 9-10 wherein the labelling substance is an enzyme.

12. The kit according to claim 11 which further comprises a substrate solution.

13. The kit according to any one of preceding claims 9-12 wherein the liposome contains a lipopolysaccharide as a part of its constituents.

## Patentansprüche

1. Methode zur Bestimmung eines Antistreptolysin-O-Werts in einer Probe, umfassend:
Zusetzen zu der Probe, die Antistreptolysin-O enthält, von:
(i) eine Lösung, enthaltend eine markierende Substanz verkapselnde Liposomen, und
(ii) eine Lösung, enthaltend Streptolysin-O; sowie
Messen der Menge der markierenden Substanz, freigesetzt durch Lyse der Liposomenmembran infolge der lytischen Aktivität des Streptolysin-O, wobei die Liposomen nach einer Detergens-Entfernungsmethode hergestellt werden.

2. Methode nach Anspruch 1, worin die Liposomen als Membranbestandteil mindestens ein eine ungesättigte Fettsäure aufweisendes Phospholipid aufweisen sowie ein Lipid mit einer negativen Ladung.

3. Methode nach einem der vorgenannten Ansprüche, worin die Liposomen zusätzlich zu der markierenden Substanz eine wasserlösliche polymere Verbindung verkapseln.

4. Methode nach einem der vorgenannten Ansprüche, worin die Liposomen zusätzlich zu der markierenden Substanz ein wasserlösliches Makromolekül verkapseln.

5. Methode nach einem der vorgenannten Ansprüche, worin die markierende Substanz ist: ein Enzym; ein Coenzym; eine lumineszierende Verbindung; eine Verbindung, die Fluoreszenz emittieren kann; ein Farbstoff, ein Zucker, eine ionische Verbindung, eine radikalische Verbindung oder eine Substanz mit Eigenschaften zur Spinmarkerierung.

6. Methode nach einem der vorgenannten Ansprüche, worin der Hauptbestandteil der Membranen der Liposome ausgewählt wird aus natürlichen Lecithinen, Phospholipiden, Mischungen von natürlichen Lecithinen und Phospholipiden, Mischungen von natürlichen Lecithinen, Phospholipiden und Cholesterinen sowie Mischungen aus natürlichen Lecithinen, Phospholipiden und Lipopolysacchariden.

7. Methode nach einem der vorgenannten Ansprüche, worin die wasserlösliche polymere Verbindung ausgewählt aus der Gruppe, bestehend aus Serumalbumin, wasserlöslicher Gelatine, Polyethylenglykol und Polyvinylalkohol.

8. Methode nach Anspruch 2 bis 7, worin das die ungesättigte Fettsäure aufweisende Phospholipid ausgewählt aus Palmityloleylphosphatidylcholin (POPC) und Dioleylphosphatidylcholin (DOPC), und worin das Lipid mit der negativen Ladung ausgewählt wird aus Lipopolysacchariden, Phosphatidylglycerinen und Phosphatidsäuren.

9. Ausrüstung zum Bestimmen des Antistreptolysin-O-Werts in einer Probe, umfassend:
(A) eine Suspension von Liposomen, hergestellt nach einer Detergens-Entfernungsmethode und Verkapseln einer markierenden Substanz und einer wasserlöslichen polymeren Verbindung; sowie
(B) eine Lösung von Streptolysin-O.

10. Ausrüstung nach Anspruch 9, worin jedes Liposom als Bestandteile seiner Membran mindestens ein eine ungesättigte Fettsäure aufweisendes Phospholipid sowie ein eine negative Ladung aufweisendes Lipid umfaßt.

11. Ausrüstung nach Anspruch 9 und 10, worin die markierende Substanz ein Enzym ist.

12. Ausrüstung nach Anspruch 11, die ferner eine Substratlösung umfaßt.

13. Ausrüstung nach Anspruch 9 bis 12, worin das Liposom als einen Teil seiner Bestandteile ein Lipopolysaccharid enthält.

## Revendications

1. Procédé pour la détermination d'une quantité d'antistreptolysine 0 dans un échantillon, qui consiste à ajouter audit échantillon contenant l'antistreptolysine 0,
(i) une solution contenant des liposomes encapsulant une substance de marquage, et
(ii) une solution contenant de la streptolysine 0, et à mesurer la quantité de ladite substance de marquage libérée par la lyse de la membrane des liposomes par l'activité lytique de la streptolysine 0, lesdits liposomes étant préparés selon un procédé d'élimination au détergent.

2. Procédé selon la revendication 1, dans lequel le liposome comprend, comme constituants de membrane, au moins un phospholipide comprenant un acide gras insaturé, et un lipide ayant une charge négative.

3. Procédé selon l'une quelconque des revendications précédentes, dans lequel le liposome encapsule un composé polymère soluble dans l'eau en plus de la substance de marquage.

4. Procédé selon l'une quelconque des revendications précédentes, dans lequel le liposome encapsule une macromolécule soluble dans l'eau en plus de la substance de marquage.

5. Procédé selon l'une quelconque des revendications précédentes, dans lequel la substance de marquage est un enzyme, un coenzyme, un composé luminescent, un composé pouvant émettre une fluorescence, un colorant, un sucre, un composé ionique, un composé radicalaire, ou une substance présentant des propriétés telles que le marquage par spin.

6. Procédé selon l'une quelconque des revendications précédentes, dans lequel le constituant principal des membranes desdits liposomes est choisi parmi les lécithines naturelles, les phospholipides, les mélanges de lécithines naturelles et de phospholipides, les mélanges de lécithines naturelles, de phospholipides et de cholestérols, et les mélanges de lécithines naturelles de phospholipides et de lipopolysaccharides.

7. Procédé selon l'une quelconque des revendications précédentes, dans lequel ledit composé polymère soluble dans l'eau est choisi parmi l'albumine sérique, la gélatine soluble dans l'eau, le polyéthylèneglycol et le poly(alcool vinylique).

8. Procédé selon l'une quelconque des revendications 2 à 7, dans lequel ledit phospholipide comprenant un acide gras insaturé est choisi parmi la palmitoyl-oléoyl-phosphatidylcholine (POPC) et la dioléoyl-phosphatidylcholine (DOPC), et dans lequel ledit lipide ayant une charge négative est choisi parmi les lipopolysaccharides, les phosphatidylglycérols et les acides phosphatidiques.

9. Trousse pour déterminer la quantité d'antistreptolysine 0 dans un échantillon, qui comprend (A) une suspension de liposomes préparée par un procédé d'élimination au détergent et encapsulant une substance de marquage et un composé polymère soluble dans l'eau, et (B) une solution de streptolysine 0.

10. Trousse selon la revendication 9, dans laquelle chaque liposome comprend, comme constituants de sa membrane, au moins un phospholipide comprenant un acide gras insaturé et un lipide ayant une charge négative.

11. Trousse selon l'une quelconque des revendications 9 et 10 dans laquelle la substance de marquage est une enzyme.

12. Trousse selon la revendication 11, qui comprend en outre une solution de substrat.

13. Trousse selon l'une quelconque des revendications 9 à 12, dans laquelle le liposome contient un lipopolysaccharide comme partie de ses constituants.
